(19) Europäisches Patentamt
European Patent Office
Office européen des brevets

(11) EP 0 815 844 B1

(12) FASCICULE DE BREVET EUROPEEN

(45) Date de publication et mention de la délivrance du brevet:
**25.11.1998 Bulletin 1998/48**

(51) Int. Cl.$^6$: **A61K 7/48**, A61K 7/06

(21) Numéro de dépôt: **97401254.4**

(22) Date de dépôt: **04.06.1997**

# (54) Composition à usage topique sous forme d'émulsion huile-dans-eau sans tensio-actif contenant un poly(acide 2-acrylamido 2-méthylpropane sulfonique) réticulé et neutralisé

Zusammensetzung in Form einer Öl-in-Wasser Emulsion ohne grenzflächenaktiven Stoff zur topischen Anwendung, die ein vernetztes und neutralisiertes Poly(2-acrylamido-2-methylpropan-sulfonsäure)-Polymer enthält

Topical surfactant-free oil-in-water emulsion containing a crosslinked neutralized polymer of 2-acrylamide-2-methylpropane sulfonic acid

(84) Etats contractants désignés:
**DE ES FR GB IT**

(30) Priorité: **28.06.1996 FR 9608109**

(43) Date de publication de la demande:
**07.01.1998 Bulletin 1998/02**

(73) Titulaire: **L'OREAL**
**75008 Paris (FR)**

(72) Inventeurs:
- **Sebillotte-Arnaud, Laurence**
**94240 L'Hay Les Roses (FR)**
- **Lorant, Raluca**
**94320 Thiais (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**US-A- 5 114 706**

- **DATABASE WPI Week 8329 Derwent Publications Ltd., London, GB; AN 83714752 XP002029282 "Surfactant free-emulsion cosmetics-comprising water, oil component and emulsion and consisting of water-insoluble and water-absorbable polymer" & JP 58 099 407 A (KANEBO LTD) 13 juin 1983**
- **DATABASE WPI Week 9547 Derwent Publications Ltd., London, GB; AN 95354385 XP002029283 "high stability cyclodextrin emulsion containing oil and alkyl-modified carboxy-vinyl polymer, and is free from surfactant" & JP 07 241 457 A (SHISEIDO CO LTD) 19 septembre 1995**

EP 0 815 844 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente demande concerne une composition cosmétique et/ou dermatologique sous forme d'émulsion huile-dans-eau sans tensio-actif contenant au moins un polymère poly(acide 2-acrylamido 2-méthylpropane sulfonique) réticulé et neutralisé à au moins 90%.

Pour diverses raisons liées en particulier à un meilleur confort d'utilisation (douceur, émollience et autres), les compositions cosmétiques ou dermatologiques actuelles se présentent le plus souvent sous la forme d'une émulsion du type huile-dans-eau (c'est-à-dire un support constitué d'une phase continue dispersante aqueuse et d'une phase discontinue dispersée huileuse) ou une émulsion du type eau-dans-huile (c'est-à-dire un support constitué d'une phase continue dispersante grasse et d'une phase discontinue dispersée aqueuse). Les émulsions huile-dans-eau sont les plus demandées dans le domaine de la cosmétique du fait qu'elles apportent sur la peau à l'application un toucher plus doux, moins gras et plus léger que les systèmes d'émulsions eau-dans-huile.

Les émulsions sont stabilisées généralement par des tensio-actifs émulsionnants du type huile-dans-eau ou du type eau-dans-huile qui grâce à leur structure amphiphiles se placent à l'interface huile/eau et stabilisent ainsi les gouttelettes dispersées. Ces molécules amphiphiles présentent cependant l'inconvénient d'être pénétrantes et irritantes pour la peau, les yeux et le cuir chevelu. D'autre part, leur présence à de fortes concentration conduit à des effets anti-cosmétiques tels que des touchers rêches, des touchers collants ou poisseux.

Les formulateurs d'émulsions recherchent constamment à réduire la teneur en tensio-actif afin d'améliorer leur innocuité vis-à-vis de la peau, des yeux et du cuir chevelu et améliorer leurs propriétés cosmétiques. La principale difficulté à laquelle ils sont généralement confrontée est d'obtenir des émulsions stables. L'objectif de l'invention est de pouvoir réaliser des émulsions huile-dans-eau stables ne contenant pas de tensio-actif émulsionnant et présentant de bonnes propriétés cosmétiques.

On connaît dans l'état de la technique des polymères poly(acide 2-acrylamido 2-méthylpropane sulfonique) comme les produits commerciaux COSMEDIA HSP1160 de la société HOECHST et RHEOTIK 8011 de la société HENKEL. Ils sont utilisés comme agents épaississants et/ou gélifiants dans de nombreuses formulations cosmétiques.

Ces polymères ne permettent pas de stabiliser des émulsions huile-dans-eau ne contenant pas de tensio-actif.

La demanderesse a découvert de façon inattendue une nouvelle famille de polymères poly(acide 2-acrylamido 2-méthylpropane sulfonique) permettant de réaliser des émulsions huile-dans-eau stables ne contenant pas de tensio-actif.

Ces polymères permettent également de préparer des émulsions huile-dans-eau, dans une large gamme de pH, dont la viscosité reste stable dans le temps à température ambiante ou à des températures plus élevées.

Ils permettent en outre de réaliser des produits homogènes, non-coulants, non-filants, doux et glissants à l'application et stables à la conservation.

La présente invention concerne une composition cosmétique et/ou dermatologique sous forme d'émulsion huile-dans-eau, caractérisée par le fait qu'elle contient au moins un polymère poly(acide 2-acrylamido 2-méthylpropane sulfonique) réticulé et neutralisé à au moins 90% et qu'elle ne contient pas de tensioactif émulsionnant.

Les polymères poly(acide 2-acrylamido 2-méthylpropane sulfonique) réticulés et pratiquement ou totalement neutralisés, conformes à l'invention, sont hydrosolubles ou gonflables dans l'eau. Ils sont en général caractérisés par le fait qu'ils comprennent, distribués de façon aléatoire :

a) de 90 à 99,9% en poids de motifs de formule générale (1) suivante :

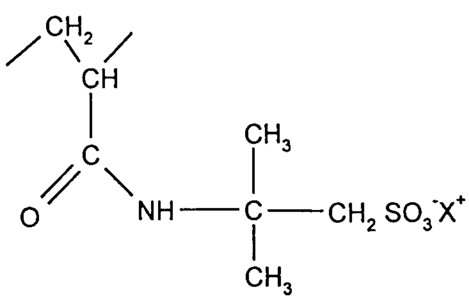

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

De façon préférentielle, les polymères de l'invention comportent un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

Les polymères selon l'invention plus particulièrement préférés comprennent de 98 à 99,5 % en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Les monomères de réticulation ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyléther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles-liaisons oléfiniques sont plus particulièrement choisis parmi ceux répondant à la formule générale (2) suivante :

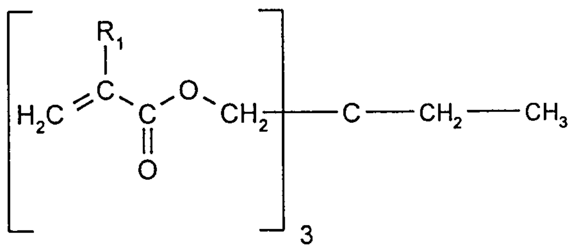 (2)

dans laquelle $R_1$ désigne un atome d'hydrogène ou un alkyle en $C_1$-$C_4$ et plus particulièrement méthyle (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec:

M désignant la masse en grammes de la macromolécule non-dissoute ;
$N_A$ désignant le nombre d'Avogadro ;
$V_1$ désignant le volume spécifique du solvant ;
$V_2$ désignant le volume spécifique de la macromolécule ;
d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution , on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al, Macromolecules, 1995, 28,4914-4919.

Les polymères particulièrement préférés sont ceux présentant une viscosité mesurée au viscosimètre BROOKFIELD, mobile 4, à une vitesse de rotation de 100 tours/minutes dans une solution d'eau à 2 % et à 25 °C supérieure ou égale à 1000 mPa • s et plus préférentiellement allant de 5000 à 40.000 mPa • s et plus particulièrement de 6500 à 35.000 mPa • s.

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol;
(b) on neutralise la solution ou la dispersion de monomère obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Un autre objet de l'invention consiste en des compositions cosmétiques ou dermatologiques contenant dans un milieu cosmétiquement acceptable au moins un poly(acide 2-acrylamido 2-méthylpropane sulfonique) réticulé et neutralisé à au moins 90% tel que décrit précédemment.

Les poly(acide 2-acrylamido 2-méthylpropane sulfonique) réticulés, pratiquement ou totalement neutralisés sont présents dans les compositions cosmétiques ou dermatologiques de l'invention dans des concentrations allant préférentiellement de 0,01 à 20% en poids par rapport au poids total de la composition et plus préférentiellement de 0,1 à 10% en poids.

Les compositions de l'invention contiennent un milieu cosmétiquement ou dermatologiquement acceptable, c'est-à-dire un milieu compatible avec toutes les matières kératiniques telles que la peau, les ongles, les muqueuses et les cheveux ou toute autre zone cutanée du corps.

Les compositions contiennent de préférence un milieu aqueux cosmétiquement et/ou dermatologiquement acceptable. Elles présentent un pH pouvant aller de préférence de 1 à 13 et plus préférentiellement de 2 à 12.

Les compositions selon l'invention peuvent contenir en plus de l'eau et de la phase grasse un ou plusieurs solvants organiques cosmétiquement et/ou dermatologiquement acceptables (tolérance, toxicologie et toucher acceptables).

Les solvants organiques peuvent représenter de 5 % à 98 % du poids total de la composition. Ils peuvent être choisis dans le groupe constitué par les solvants organiques hydrophiles, les solvants organiques lipophiles ou leurs mélanges.

Parmi les solvants organiques hydrophiles, on peut citer par exemple des mono-alcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer des polyols tels que des dérivés de polypropylène glycol (PPG) comme les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

La phase grasse des compositions selon l'invention représente de préférence, de 0 % à 50 % du poids total de la composition.

Cette phase grasse peut comporter une ou plusieurs huiles choisies de préférence dans le groupe constitué par :

- les silicones volatiles ou non-volatiles, linéaires, ramifiées ou cycliques, organo-modifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de purcellin, les isoparaffines,
- les huiles fluorées et perfluorées,
- les esters d'acides gras tels que l'huile de Purcellin.

Elle peut aussi comporter comme matière grasse un(e) ou plusieurs alcool gras, acides gras (acide stéarique) ou cires (paraffine, cires de polyéthylène, carnauba, cire d'abeilles).

De façon connue, toutes les compositions de l'invention peuvent contenir des adjuvants habituels dans les domaines cosmétique et dermatologique, d'autres gélifiants et/ou épaississants classiques ; des polymères ; des agents hydratants ; des émollients ; des filtres solaires ; des actifs hydrophiles ou lipophiles comme des céramides ; des agents anti-radicaux libres ; des répulsifs pour insectes ; des agents amincissants ; des bactéricides ; des séquestrants ; des antipelliculaires ; des antioxydants ; des conservateurs ; des agents alcanisants ou acidifiants ; des parfums ; des charges ; des matières colorantes. Les quantités de ces différents adjuvants sont celles classiquement utilisées dans les domaines considérés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de sérum, de lait, de crèmes plus ou moins onctueuse, de pâte. Ces compositions sont préparées selon les méthodes usuelles.

Les compositions selon l'invention peuvent être utilisées pour la préparation de ou comme produits rincés ou comme produits non-rincés capillaires notamment pour le soin et le conditionnement des cheveux.

Les compositions de l'invention peuvent être également utilisées pour la préparation d'un ou comme produit de soin de la peau, des cheveux, du cuir chevelu, des cils, des sourcils, des ongles ou des muqueuses tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions de l'invention peuvent être également utilisées pour la préparation d'un ou comme, produit antisolaire.

Les compositions peuvent être utilisées pour la préparation de ou comme, produits pour le maquillage tels que des crèmes pour le visage, des fonds de teint.

Un autre objet de l'invention est un procédé de traitement non-thérapeutique cosmétique de la peau, du cuir chevelu, des cheveux, des cils, des sourcils, des ongles ou des muqueuses, caractérisé par le fait qu'on applique sur le support kératinique une composition telle que définie ci-dessus, selon la technique d'utilisation habituelle de cette composition. Par exemple : application de crèmes, de gels, de sérums, de lotions, de laits sur la peau, le cuir chevelu et/ou les muqueuses.

Les exemples suivants illustrent l'invention sans présenter un caractère limitatif.

**EXEMPLE DE PREPARATION A**

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant viscosité mesurée au viscosimètre BROOKFIELD, mobile 4, à une vitesse de rotation de 100 tours/minutes dans une solution d'eau à 2 % et à 25 °C allant de 15000 mPa • s à 35.000 mPa • s. La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

**EXEMPLE DE PREPARATION B**

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minutes dans une solution d'eau à 2 % et à 25 °C de l'ordre de 7000 mPa • s.

Le rayon hydrodynamique du polymère obtenu dans une solution aqueuse déterminé par diffusion de la lumière dynamique est de 160 nm.

**ESSAIS COMPARATIFS**

On étudie l'aspect macroscopique et l'aspect microscopique d'émulsions huile-dans-eau (1) selon l'invention, ne contenant pas de tensio-actif, épaissies par un polymère Poly(acide 2-acrylamido 2-méthylpropane sulfonique) réticulé et neutralisé par de l'ammoniaque, préparé selon le procédé de l'exemple de préparation A de viscosité de l'ordre de 16.000 cps dans une solution d'eau à 2% et à 25°C.

On compare ces compositions à des émulsions huile-dans-eau (2) selon l'art antérieur contenant comme épaississant un polymère Poly(acide 2-acrylamido 2-méthylpropane sulfonique) non réticulé tel que le produit commercial COSMEDIA HSP1160 vendu par HENKEL.

Les compositions (1) et (2) ont comme formulation :

**COMPOSITION (1)**

| Phase grasse | |
|---|---|
| - Huile d'amande douce | 7,0 g |
| - Cyclométhicone | 5,0 g |

| Phase aqueuse | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPa • s dans une solution d'eau à 2% et à 25°C | 0,5-2 g MA |
| - Eau distillée qs pH 2 à 7 | 100 g |

**COMPOSITION (2)**

| Phase grasse | |
|---|---|
| - Huile d'amande douce | 7,0 g |
| - Cyclométhicone | 5,0 g |

| Phase aqueuse | |
|---|---|
| - Poly(acide 2-acrylamido 2-méthylpropane sulfonique) non-réticulé et neutralisé par de la triéthanolamine vendu sous le nom COSMEDIA HSP1160 | 0,5-11g MA |
| - Eau distillée qs pH 2 à 7 | 100 g |

On obtient avec les compositions (1), des gels ou des crèmes, plus ou moins épaissis, stables, homogènes, non-collants et non-filants. On observe au microscope des dispersions stables, fines, régulières et homogènes des gouttelettes d'huile.

On obtient avec les compositions (2), des gels fluides, instables, hétérogènes, poisseux, filants et collants même à des concentrations élevées en polymère épaississant (11% en matière active). On observe au microscope des dispersions instables, grossières, irrégulières et hétérogènes des gouttelettes d'huile.

**EXEMPLE 1 : Crème hydratante**

| Phase grasse | |
|---|---|
| - Huile d'amande douce | 7,0 g |
| - Cyclométhicone | 5,0 g |

| Phase aqueuse | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPa • s dans une solution d'eau à 2% et à 25°C | 1,5 g MA |
| - Glycérine | 7,0 g |
| - Conservateur qs | |
| - Eau distillée qs pH 6 | 100 g |

On obtient une crème gélifiée, blanche, brillante et homogène.

**<u>EXEMPLE 2 :</u> Crème douce desquamante acide**

| Phase grasse | |
|---|---|
| - Huile d'amande douce | 10,0 g |

| Phase aqueuse | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 cps dans une solution d'eau à 2% et à 25°C | 2 g MA |
| - Acide malique | 1,0 g |
| - Acide tartrique | 1,0 g |
| - Conservateur qs | |
| - Eau distillée qs pH 3,5 | 100 g |

On obtient une crème épaisse, blanche, brillante et homogène.

**<u>EXEMPLE 3 :</u> Fluide fraîcheur pour peaux grasses**

| Phase grasse | |
|---|---|
| - Silicone volatile | 5,0 g |

| Phase aqueuse | |
|---|---|
| - Poly(acide 2-acrylamido 2-méthylpropane sulfonique) réticulé et neutralisé par de l'ammoniaque, préparé selon le procédé de préparation A de viscosité de l'ordre de 16.000 mPa • s dans une solution d'eau à 2% et à 25°C | 0,5 g MA |

(suite)

| Phase aqueuse | |
|---|---|
| - Alcool éthylique | 20,0 g |
| - Conservateur qs | |
| - Eau distillée qs pH 6 | 100 g |

On obtient un fluide translucide, stable et homogène.

**EXEMPLE 4 : Emulsion fluide dépigmentante acide**

| Phase grasse | |
|---|---|
| - Huile d'avocat | 8,0 g |

| Phase aqueuse | |
|---|---|
| - Poly(acide 2-acrylamido 2-méthylpropane sulfonique) réticulé et neutralisé par de l'ammoniaque, préparé selon le procédé de l'exemple de préparation A de viscosité de l'ordre de 16.000 mPa • s dans une solution d'eau à 2% et à 25°C | 0,7 g MA |
| - Acide kojique | 1,0 g |
| - Acide téréphtalidène-3,3' di-camphosulfonique-10,10' | 0,7 g |
| - Triéthanolamine qs | |
| - Conservateur qs | |
| - Eau distillée qs pH 3 | 100 g |

On obtient un fluide d'aspect émulsionné, stable et homogène.

**EXEMPLE 5 : Gel délassant pour jambes lourdes**

| Phase grasse | |
|---|---|
| - Fraction liquide de beurre de karité | 3 g |
| - Cyclométhicone | 3 g |

| Phase aqueuse | |
|---|---|
| - Poly(acide 2-acrylamido 2-méthylpropane sulfonique) réticulé et neutralisé par de l'ammoniaque, préparé selon le procédé de l'exemple de préparation B de viscosité de l'ordre de 7.000 mPa • s dans une solution d'eau à 2% et à 25°C | 1,5 g MA |
| - Alcool éthylique à 96° dans l'eau | 20 g |
| - Glycérine | 3 g |

(suite)

| Phase aqueuse | |
|---|---|
| - Propylène glycol | 3 g |
| - Menthol | 0,3 g |
| - Camphre | 0,2 g |
| - Eau déminéralisée qs pH 5 | 100 g |

On obtient un gel blanc, brillant, homogène et lisse.

**Revendications**

**1.** Composition cosmétique et/ou dermatologique sous forme d'émulsion huile-dans-eau, caractérisée par le fait qu'elle contient au moins un polymère poly(acide 2-acrylamido 2-méthylpropane sulfonique) réticulé et neutralisé à au moins 90% et qu'elle ne contient pas de tensio-actif.

**2.** Composition selon la revendication 1, caractérisée par le fait que les poly(acide 2-acrylamido 2-méthylpropane sulfonique) réticulés et neutralisés à au moins 90% comprennent, distribués de façon aléatoire :

a) de 90 à 99,9% en poids de motifs de formule générale (1) suivante :

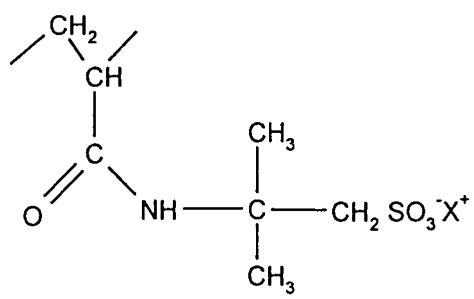

(1)

dans laquelle $X^+$ désigne un cation ou un mélange de cations, au plus 10% mol des cations $X^+$ pouvant être des protons $H^+$ ;
b) de 0,01 à 10% en poids de motifs réticulants provenant d'au moins un monomère ayant au moins deux doubles-liaisons oléfiniques ; les proportions en poids étant définis par rapport au poids total du polymère.

**3.** Composition selon la revendication 2, caractérisée par le fait que les poly(acide 2-acrylamido 2-méthylpropane sulfonique) réticulés et neutralisés à au moins 90% comportent un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

**4.** Composition selon l'une quelconque des revendications 2 et 3, caractérisée par le fait que les poly(acide 2-acrylamido 2-méthylpropane sulfonique) réticulés et neutralisés à au moins 90% comportent de 98 à 99,5 % en poids de motifs de formule (1) et de 0,2 à 2 % en poids de motifs réticulants.

**5.** Composition selon l'une quelconque des revendications 2 à 4, caractérisée par le fait que dans la formule (1) le cation $X^+$ est $NH_4^+$.

**6.** Composition selon l'une quelconque des revendications 2 à 5, caractérisée par le fait que les monomères réticulants répondent à la formule générale (2) suivante :

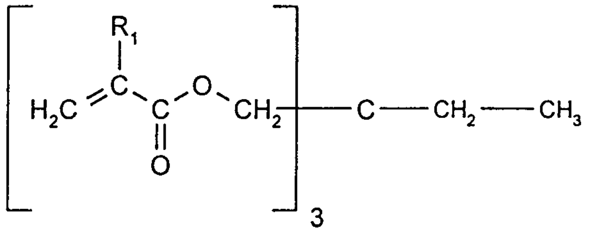

(2)

dans laquelle $R_1$ désigne hydrogène ou un alkyle en $C_1$-$C_4$.

7. Composition selon l'une quelconque des revendications 2 à 6, caractérisée par le fait que le poly(acide 2-acrylamido 2-méthylpropane sulfonique) est réticulé par le triméthylol propane triacrylate.

8. Composition selon l'une quelconque des revendications 2 à 7, caractérisée par le fait que les polymères de formule (1) présentent une viscosité mesurée au viscosimètre BROOKFIELD, mobile 4, vitesse 100 tours/minute, dans une solution d'eau à 2 % et à 25 °C, supérieure ou égale à 1000 MPa • s.

9. Composition selon l'une quelconque des revendications 2 à 7, caractérisée les polymères de formule (1) présentent une viscosité mesurée au viscosimètre BROOKFIELD, mobile 4, vitesse 100 tours/minute, dans une solution d'eau à 2 % et à 25 °C allant de 5000 à 40000 MPa • s et plus particulièrement de 6500 à 35000 MPa • s.

10. Composition selon l'une quelconque des revendications 1 à 9, caractérisée par le fait que le poly(acide 2-acrylamido 2-méthylpropane sulfonique) réticulé est présent dans des concentrations allant préférentiellement de 0,01 à 20% en poids par rapport au poids total de la composition et plus préférentiellement de 0,1 à 10% en poids.

11. Composition selon l'une quelconque des revendications 1 à 10, caractérisée par le fait qu'elle présente un pH allant de 1 à 13.

12. Composition selon l'une quelconque des revendications 1 à 11, caractérisée en ce qu'elle contient en plus au moins un solvant organique choisi dans le groupe constitué par les solvants organiques hydrophiles, les solvants organiques lipophiles ou leurs mélanges.

13. Composition selon la revendication 12, caractérisée en ce que les solvants organiques sont choisis dans le groupe constitué par les alcools mono- ou polyfonctionnels, les polyéthylène glycols éventuellement oxyéthylénés, les esters de propylène glycol, le sorbitol et ses dérivés, les di-alkyles d'isosorbide, les éthers de glycol et les éthers de propylène glycol, les esters gras.

14. Composition selon la revendication 12 ou 13, caractérisée en ce que le ou les solvants organiques représentent de 5 % à 98 % du poids total de la composition.

15. Composition selon l'une quelconque des revendications 1 à 14, caractérisée en ce que la phase grasse représente de 0 à 50% du poids total de la composition.

16. Composition selon l'une quelconque des revendications 1 à 15, caractérisée par le fait qu'elle contient en plus au moins un additif choisi dans le groupe constitué par les gélifiants et/ou épaississants classiques aqueux ou lipophiles ; des actifs hydrophiles ou lipophiles ; des conservateurs ;des antioxydants ; des parfums ; des agents hydratants ; des émollients ; des séquestrants ; des polymères ; des agents alcanisants ou acidifiants ; des charges ; des agents anti-radicaux libres ; des céramides ; des filtres solaires ; des répulsifs pour insectes; des agents amincissants ; des matières colorantes ; des bactéricides ; des antipelliculaires.

17. Utilisation de la composition selon l'une quelconque des revendications 1 à 16, pour la préparation d'un produit capillaire rincé ou non-rincé ou utilisation non-thérapeutique cosmétique de la composition selon l'une quelconque des revendications 1 à 16 comme produit capillaire rincé ou non rincé.

18. Utilisation de la composition selon l'une quelconque des revendications 1 à 16, pour la préparation d'un produit de soin pour la peau, les cheveux, le cuir chevelu, des cils, des sourcils, des ongles ou des muqueuses ou utilisation

non-thérapeutique cosmétique de la composition selon l'une quelconque des revendications 1 à 16 comme produit de soin pour la peau, les cheveux, le cuir chevelu, des cils, des sourcils, des ongles ou des muqueuses.

19. Utilisation de la composition selon l'une quelconque des revendications 1 à 16, pour la préparation d'un produit de maquillage ou utilisation non-thérapeutique cosmétique de la composition selon l'une quelconque des revendications 1 à 16 comme produit de maquillage.

20. Utilisation de la composition selon l'une quelconque des revendications 1 à 16, pour la préparation d'un produit antisolaire ou utilisation non-thérapeutique cosmétique de la composition selon l'une quelconque des revendications 1 à 16 comme produit antisolaire.

21. Procédé de traitement non-thérapeutique cosmétique de la peau, du cuir chevelu, des cheveux, des cils, des sourcils, des ongles ou des muqueuses, caractérisé par le fait qu'on applique sur le support une composition telle que définie selon l'une quelconque des revendications 1 à 16.

**Claims**

1. Cosmetic and/or dermatological composition in the form of an oil-in-water emulsion, characterized in that it contains at least one crosslinked and at least 90 % neutralized poly(2-acrylamido-2-methylpropanesulphonic acid) polymer and in that it contains no surfactant.

2. Composition according to Claim 1, characterized in that the crosslinked and at least 90 % neutralized poly(2-acrylamido-2-methylpropanesulphonic acids) comprise, randomly distributed:

   a) from 90 to 99.9 % by weight of units of general formula (1) below:

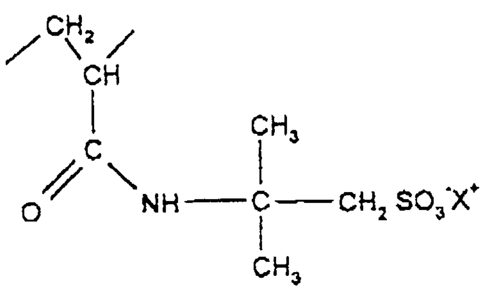

(1)

   in which $X^+$ denotes a cation or a mixture of cations, it being possible for not more than 10 mol% of the cations $X^+$ to be protons $H^+$;
   b) from 0.01 to 10 % by weight of crosslinking units originating from at least one monomer having at least two olefinic double bonds; the weight proportions being defined relative to the total weight of the polymer.

3. Composition according to Claim 2, characterized in that the crosslinked and at least 90 % neutralized poly(2-acrylamido-2-methylpropanesulphonic acids) contain a number of units of formula (1) in an amount which is sufficiently large to obtain polymer particles whose hydrodynamic volume in aqueous solution has a radius ranging from 10 to 500 nm and whose distribution is homogeneous and unimodal.

4. Composition according to either of Claims 2 and 3, characterized in that the crosslinked and at least 90 % neutralized poly(2-acrylamido-2-methylpropanesulphonic acids) contain from 98 to 99.5 % by weight of units of formula (1) and from 0.2 to 2 % by weight of crosslinking units.

5. Composition according to any one of Claims 2 to 4, characterized in that, in formula (1), the cation $X^+$ is $NH_4^+$.

6. Composition according to any one of Claims 2 to 5, characterized in that the crosslinking monomers correspond to general formula (2) below:

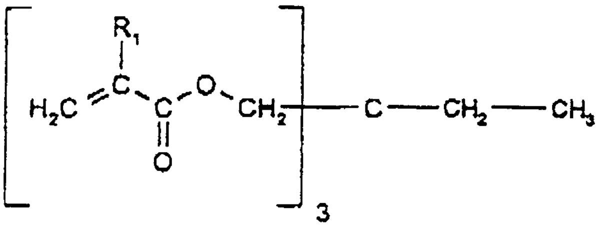

(2)

in which $R_1$ denotes hydrogen or a $C_1$-$C_4$ alkyl.

7. Composition according to any one of Claims 2 to 6, characterized in that the poly(2-acrylamido-2-methylpropanesulphonic acid) is crosslinked with trimethylolpropane triacrylate.

8. Composition according to any one of Claims 2 to 7, characterized in that the polymers of formula (1) have a viscosity, measured with a Brookfield viscometer, rotor 4, speed 100 revolutions/ minute, in an aqueous solution at a concentration of 2 % and at 25°C, of greater than or equal to 1000 mPa.s.

9. Composition according to any one of Claims 2 to 7, characterized in that the polymers of formula (1) have a viscosity, measured with a Brookfield viscometer, rotor 4, speed 100 revolutions/ minute, in an aqueous solution at a concentration of 2 % and at 25°C, ranging from 5000 to 40,000 mPa.s and more particularly from 6500 to 35,000 mPa.s.

10. Composition according to any one of Claims 1 to 9, characterized in that the crosslinked poly(2-acrylamido-2-methylpropanesulphonic acid) is present in concentrations preferably ranging from 0.01 to 20 % by weight relative to the total weight of the composition, and more preferably from 0.1 to 10 % by weight.

11. Composition according to any one of Claims 1 to 10, characterized in that it has a pH ranging from 1 to 13.

12. Composition according to any one of Claims 1 to 11, characterized in that it also contains at least one organic solvent chosen from the group consisting of hydrophilic organic solvents, lipophilic organic solvents or mixtures thereof.

13. Composition according to Claim 12, characterized in that the organic solvents are chosen from the group consisting of mono- or polyfunctional alcohols, optionally oxyethylenated polyethylene glycols, propylene glycol esters, sorbitol and derivatives thereof, di-alkyl isosorbides, glycol ethers and propylene glycol ethers, and fatty esters.

14. Composition according to Claim 12 or 13, characterized in that the organic solvent or solvents represent from 5 % to 98 % of the total weight of the composition.

15. Composition according to any one of Claims 1 to 14, characterized in that the fatty phase represents from 0 to 50 % of the total weight of the composition.

16. Composition according to any one of Claims 1 to 15, characterized in that it also contains at least one additive chosen from the group consisting of standard aqueous or lipophilic gelling agents and/or thickeners; hydrophilic or lipophilic active agents; preserving agents; antioxidants; fragrances; hydrating agents; emollients; sequestering agents; polymers; basifying or acidifying agents; fillers; anti-free-radical agents; ceramides; sunscreens; insect repellants; slimming agents; dyestuffs; bactericides; anti-dandruff agents.

17. Use of the composition according to any one of Claims 1 to 16 for the preparation of a rinse-out or leave-in hair product, or non-therapeutic cosmetic use of the composition according to any one of Claims 1 to 16 as a rinse-out or leave-in hair product.

18. Use of the composition according to any one of Claims 1 to 16 for the preparation of a product to care for the skin, the hair, the scalp, the eyelashes, the eyebrows, the nails or the mucous membranes, or non-therapeutic cosmetic use of the composition according to any one of Claims 1 to 16 as a care product for the skin, the hair, the scalp, the

eyelashes, the eyebrows, the nails or mucous membranes.

19. Use of the composition according to any one of Claims 1 to 16 for the preparation of a make-up product, or non-therapeutic cosmetic use of the composition according to any one of Claims 1 to 16 as a make-up product.

20. Use of the composition according to any one of Claims 1 to 16 for the preparation of an antisun product, or non-therapeutic cosmetic use of the composition according to any one of Claims 1 to 16 as an antisun product.

21. Process for the cosmetic, non-therapeutic treatment of the skin, the scalp, the hair, the eyelashes, the eyebrows, the nails or the mucous membranes, characterized in that a composition as defined according to any one of Claims 1 to 16 is applied to the support.

**Patentansprüche**

1. Kosmetische und/oder dermatologische Zusammensetzungen in Form einer Öl-in-Wasser-Emulsion, dadurch gekennzeichnet, daß sie mindestens ein vernetztes und zu mindestens 90 % neutralisiertes Poly(2-acrylamido-2-methylpropan-sulfonsäure)-Polymer und keinen grenzflächenaktiven Stoff enthalten.

2. Zusammensetzungen nach Anspruch 1, dadurch gekennzeichnet, daß die vernetzten und zu mindestens 90 % neutralisierten Poly(2-acrylamido-2-methylpropan-sulfonsäure)-Polymere statistisch verteilt enthalten:

   a) 90 bis 99,9 Gew.-% Einheiten der folgenden allgemeinen Formel (1)

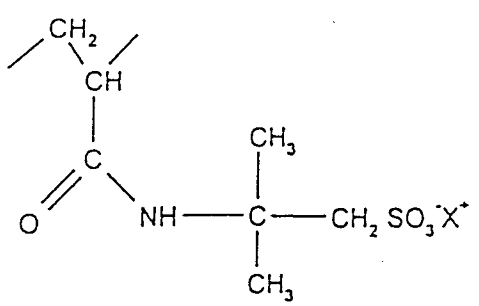

(1),

   worin $X^+$ ein Kation oder ein Gemisch von Kationen bedeutet, wobei höchstens 10 Mol-% der Kationen $X^+$ Protonen $H^+$ sein können;
   b) 0,01 bis 10 Gew.-% vernetzende Einheiten, die von mindestens einem Monomer stammen, das mindestens zwei olefinische Doppelbindungen aufweist; die Mengenanteile in Gewichtsprozent sind bezogen auf das Gesamtgewicht des Polymers angegeben.

3. Zusammensetzungen nach Anspruch 2, dadurch gekennzeichnet, daß die vernetzten und zu mindestens 90 % neutralisierten Poly(2-acrylamido-2-methylpropan-sulfonsäure)-Polymere Einheiten der Formel (1) in einer Anzahl enthalten, die ausreichend hoch ist, um Polymerpartikel herzustellen, deren hydrodynamisches Volumen in wäßriger Lösung einen Radius im Bereich von 10 bis 500 nm aufweist und deren Verteilung homogen und unimodal ist.

4. Zusammensetzungen nach Anspruch 2 oder 3, dadurch gekennzeichnet, daß die vernetzten und zu mindestens 90 % neutralisierten Poly(2-acrylamido-2-methylpropan-sulfonsäure)-Polymere 98 bis 99,5 Gew.-% Einheiten der Formel (1) und 0,2 bis 2 Gew.-% Vernetzungseinheiten enthalten.

5. Zusammensetzungen nach einem der Ansprüche 2 bis 4, dadurch gekennzeichnet, daß in der Formel (1) das Kation $X^+$ $NH_4^+$ bedeutet.

6. Zusammensetzungen nach einem der Ansprüche 2 bis 5, dadurch gekennzeichnet, daß die Monomere zur Vernetzung der folgenden allgemeinen Formel (2) entsprechen:

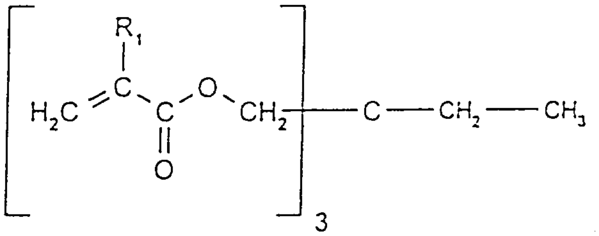

(2) ,

worin $R_1$ Wasserstoff oder $C_{1-4}$-Alkyl bedeutet.

7. Zusammensetzungen nach einem der Ansprüche 2 bis 6, dadurch gekennzeichnet, daß das Poly(2-acrylamido-2-methylpropan-sulfonsäure)-Polymer mit Trimethylolpropantriacrylat vernetzt ist.

8. Zusammensetzungen nach einem der Ansprüche 2 bis 7, dadurch gekennzeichnet, daß die Polymere der Formel (1) eine mit einem BROOKFIELD-Viskosimeter (bewegliches Teil 4) bei einer Rotationsgeschwindigkeit von 100 U/min in einer 2%igen wäßrigen Lösung bei 25 °C bestimmte Viskosität von mindestens 1 000 mPa • s aufweisen.

9. Zusammensetzungen nach einem der Ansprüche 2 bis 7, dadurch gekennzeichnet, daß die Polymere der Formel (1) eine mit einem BROOKFIELD-Viskosimeter (bewegliches Teil 4) bei einer Rotationsgeschwindigkeit von 100 U/min in einer 2%igen wäßrigen Lösung bei 25 °C bestimmte Viskosität im Bereich von 5 000 bis 40 000 mPa • s und insbesondere 6 500 bis 35 000 mPa • s aufweisen.

10. Zusammensetzungen nach einem der Ansprüche 1 bis 9, dadurch gekennzeichnet, daß die vernetzte Poly(2-acrylamido-2-methylpropan-sulfon-säure) in Konzentrationen vorzugsweise im Bereich von 0,01 bis 20 Gew.-%, bezogen auf das Gesamtgewicht der Zusammensetzung, und noch bevorzugter im Bereich von 0,1 bis 10 Gew.-% vorliegt.

11. Zusammensetzungen nach einem der Ansprüche 1 bis 10, dadurch gekennzeichnet, daß sie einen pH-Wert im Bereich von 1 bis 13 aufweisen.

12. Zusammensetzungen nach einem der Ansprüche 1 bis 11, dadurch gekennzeichnet, daß sie ferner mindestens ein organisches Lösungsmittel enthalten, das unter den hydrophilen organischen Lösungsmitteln, den lipophilen organischen Lösungsmitteln oder deren Gemischen ausgewählt ist.

13. Zusammensetzungen nach Anspruch 12, dadurch gekennzeichnet, daß die organischen Lösungsmittel unter den einwertigen oder mehrwertigen Alkoholen, den gegebenenfalls ethoxylierten Polyethylenglykolen, den Propylenglykolestern, Sorbit und seinen Derivaten, den Dialkylisosorbiden, den Glykolethern, den Propylenglykolethern und den Fettsäureestern ausgewählt sind.

14. Zusammensetzungen nach Anspruch 12 oder 13, dadurch gekennzeichnet, daß das oder die organischen Lösungsmittel 5 bis 98 % des Gesamtgewichts der Zusammensetzung ausmachen.

15. Zusammensetzungen nach einem der Ansprüche 1 bis 14, dadurch gekennzeichnet, daß die Fettphase 0 bis 50 % des Gesamtgewichts der Zusammensetzung ausmacht.

16. Zusammensetzungen nach einem der Ansprüche 1 bis 15, dadurch gekennzeichnet, daß sie ferner mindestens einen Zusatzstoff enthalten, der ausgewählt ist unter: herkömmlichen wäßrigen oder lipophilen Gelbildnern oder Verdickungsmitteln, hydrophilen oder lipophilen Wirkstoffen, Konservierungsmittel, Antioxidantien, Parfums, Hydratisierungsmitteln, Emollentien, Maskierungsmitteln, Polymeren, Mitteln zum Ansäuern oder Alkalischmachen, Füllstoffen, Mitteln gegen freie Radikale, Ceramiden, Sonnenschutzfiltern, insektenabwehrenden Mitteln, schlankermachenden Mitteln, Färbemitteln, Bakteriziden und Mitteln gegen Schuppen.

17. Verwendung der Zusammensetzung nach einem der Ansprüche 1 bis 16 zur Herstellung eines Produktes für das

Haar, das ausgespült oder nicht ausgespült wird, oder nicht therapeutische kosmetische Verwendung der Zusammensetzung nach einem der Ansprüche 1 bis 16 als Produkt für das Haar, das ausgespült oder nicht ausgespült wird.

**18.** Verwendung der Zusammensetzung nach einem der Ansprüche 1 bis 16 zur Herstellung eines Produktes zur Pflege der Haut, der Haare, der Kopfhaut, der Wimpern, der Augenbrauen, der Nägel oder der Schleimhäute, oder nicht therapeutische kosmetische Verwendung der Zusammensetzung nach einem der Ansprüche 1 bis 16 als Produkt zur Pflege der Haut, der Haare, der Kopfhaut, der Wimpern, der Augenbrauen, der Nägel oder der Schleimhäute.

**19.** Verwendung der Zusammensetzung nach einem der Ansprüche 1 bis 16 zur Herstellung eines Produktes zum Schminken oder nicht therapeutische kosmetische Verwendung der Zusammensetzung nach einem der Ansprüche 1 bis 16 als Produkt zum Schminken.

**20.** Verwendung der Zusammensetzung nach einem der Ansprüche 1 bis 16 zur Herstellung eines Sonnenschutzmittels oder nicht therapeutische kosmetische Verwendung der Zusammensetzung nach einem der Ansprüche 1 bis 16 als Sonnneschutzmittel.

**21.** Nicht therapeutisches kosmetisches Verfahren zur Behandlung der Haut, der Kopfhaut, der Haare, der Wimpern, der Augenbrauen, der Nägel oder der Schleimhäute, dadurch gekennzeichnet, daß auf den Träger eine Zusammensetzung nach einem der Ansprüche 1 bis 16 aufgetragen wird.